(19) Europäisches Patentamt
European Patent Office
Office européen des brevets

(11) Veröffentlichungsnummer : **0 051 816**
**B1**

(12) # EUROPÄISCHE PATENTSCHRIFT

(45) Veröffentlichungstag der Patentschrift : **02.05.84**

(21) Anmeldenummer : **81109189.1**

(22) Anmeldetag : **29.10.81**

(51) Int. Cl.³ : **C 07 D405/12**, C 07 D409/12, A 61 K 31/41// C07D257/04 ,(C07D405/12, 257/04, 307/52),(C07D409/12, 257/04, 333/20)

(54) **Basisch substituierte 5-Phenyltetrazole, Verfahren zu ihrer Herstellung und ihre Verwendung als Heilmittel.**

(30) Priorität : **06.11.80 DE 3041812**

(43) Veröffentlichungstag der Anmeldung : **19.05.82 Patentblatt 82/20**

(45) Bekanntmachung des Hinweises auf die Patenterteilung : **02.05.84 Patentblatt 84/18**

(84) Benannte Vertragsstaaten : **AT BE CH DE FR GB IT LI LU NL SE**

(56) Entgegenhaltungen :
**AT-B- 310 744**
**DE-A- 1 815 922**

**Die Akte enthält technische Angaben, die nach dem Eingang der Anmeldung eingereicht wurden und die nicht in dieser Patentschrift enthalten sind.**

(73) Patentinhaber : **HOECHST AKTIENGESELLSCHAFT**
**Postfach 80 03 20**
**D-6230 Frankfurt am Main 80 (DE)**

(72) Erfinder : **Sturm, Karl, Dr.**
**Berndesallee 64**
**D-6501 Heidesheim (DE)**
Erfinder : **Muschaweck, Roman, Dr.**
**Helmchenweg 39**
**D-6230 Frankfurt am Main 80 (DE)**
Erfinder : **Hropot, Max, Dr.**
**Friedrich-Stolz-Strasse 13**
**D-6093 Flörsheim am Main (DE)**

**EP 0 051 816 B1**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

Basisch substituierte 5-Phenyltetrazole, Verfahren zu ihrer Herstellung und ihre Verwendung als Heilmittel

Gegenstand der Erfindung sind Verbindungen der Formel I, die der Gruppe der 5-Phenyltetrazole zuzuordnen sind, sowie deren physiologisch verträgliche Salze.

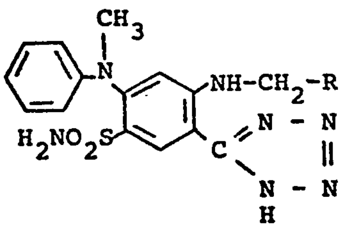

(I)

In der Formel I bedeutet R einen Furyl-, Thienyl- oder Phenylrest, vorzugsweise den 2-Furyl- oder 2-Thienylrest.

Als Kationen der beanspruchten Salze I kommen für die therapeutische Verwendung in erster Linie Natrium-, Kalium-, Ammonium- und substituierte Ammoniumionen in Frage. Besondere Bedeutung haben auch die Salze aus I und einem basischen Arzneimittel wie Antihypertensiva, β-Blockern oder kaliumretinierenden Substanzen.

Gegenstand der Erfindung ist ferner ein Verfahren zur Herstellung von Verbindungen der Formel I, das dadurch gekennzeichnet ist, daß man eine Verbindung der Formel II

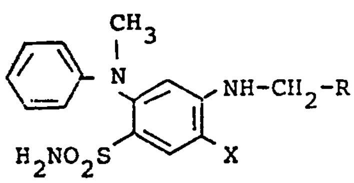

(II)

in der X eine Nitril-, Imidoester-, Amidin- oder Amidrazongruppe bedeutet, mit Stickstoffwasserstoffsäure bzw. salpetriger Säure oder einem reaktiven Derivat einer dieser Säuren umsetzt.

Ein bevorzugtes technisches Verfahren ist die Umsetzung eines Nitrils (II mit X = CN) mit Stickstoffwasserstoffsäure. Diese Umsetzung erfolgt durch einfaches Erhitzen der Reaktionskomponenten in einem inerten Lösungsmittel, vorzugsweise Dimethylformamid. Anstelle der Stickstoffwasserstoffsäure verwendet man vorteilhaft die einfacher zu handhabenden Alkalisalze, beispielsweise das Natriumazid, und aktiviert diese im Reaktionsgemisch durch eine schwache Säure oder eine schwach sauer wirkende Verbindung wie Ammoniumchlorid. Analog kann die Umsetzung einer Imidoester- oder Amidingruppierung zum Tetrazol erfolgen, während eine Amidrazongruppe mittels salpetriger Säure oder deren Salzen in den Tetrazolring übergeführt wird.

Die bevorzugt als Ausgangsmaterial verwendeten Nitrile der allgemeinen Formel III können in einfacher Weise beispielsweise gemäß dem nachstehenden Formelschema hergestellt werden.

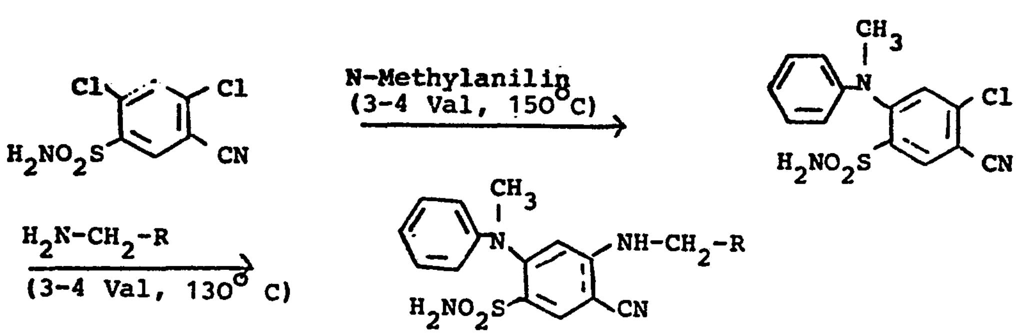


(III)

Die Verfahrensprodukte können sowohl in freier Form als auch in Form ihrer Salze isoliert werden. Besonders vorteilhaft ist die Isolierung der Natrium- oder Kaliumsalze, die bei Raumtemperatur in Wasser nur wenig, in der Wärme sehr gut löslich sind.

Zur Überführung in ein Ammoniumsalz fällt man vorteilhaft das freie Tetrazol aus einer wäßrigen Lösung des Alkalisalzes mit verdünnter Salzsäure bei pH 3 aus und kombiniert es dann in einem

geeigneten Lösungsmittel mit der äquimolaren Menge des gewünschten Amins. Besondere pharmakologische Bedeutung haben die Salze der erfindungsgemäßen Verbindungen mit basischen kaliumretinierenden Verbindungen wie beispielsweise Amilorid oder Triamteren oder mit basischen Antihypertensiva wie beispielsweise Clonidin, Dihydralazin oder β-Blockern.

Die erfindungsgemäßen Verbindungen sind ausgezeichnete Salidiuretika vom Furosemid-Typ. Gegenüber den in DE-A-1 815 922 und AT-B-310 744 beschriebenen Salidiuretika mit Tetrazolstruktur zeichnen sie sich durch höhere Potenz, bessere Resorbierbarkeit und eine uricosurische Wirkungskomponente aus.

## Beispiele

### Beispiel 1

5-[2-Furfurylamino-4-(N-methylanilino)-5-sulfamoylphenyl]-tetrazolyl-natrium

38,3 g 2-Furfurylamino-4-(N-methylanilino)-5-sulfamoylbenzonitril (0,1 Mol) vom Schmp. 205 °C (aus Methanol) werden zusammen mit 13,0 g Natriumazid und 11,0 g Ammoniumchlorid in 0,6 l Dimethylformamid 3 Stunden bei 110 °C gerührt. Nachfolgend zieht man das Dimethylformamid im Vakuum ab und nimmt den Eindampfrückstand in 0,3 l 1n NaOH auf. Die Lösung wird mit Aktivkohle entfärbt und dann mit 2n HCl auf ph 8,0 eingestellt. Nach Stehen über Nacht bei 10 °C saugt man die Fällung ab und kristallisiert das Endprodukt nochmals aus Wasser um. Nach Waschen mit Isopropanol wird bei 100 °C getrocknet.

Ausbeute : 36,5 g (81 % d. Th.), Schmp. 221 °C (u. Zers.)

### Beispiel 2

5-[2-(2-Thienylmethylamino)-4-(N-methylanilino)-5-sulfamoylphenyl]-tetrazolyl-natrium

39,9 g 2-(2-Thienylmethylamino)-4-(N-methylanilino)-5-sulfamoylbenzonitril (0,1 Mol) vom Schmp. 182 °C (aus Meth.) werden analog Beispiel 1 mit $HN_3$ kondensiert und das Endprodukt wie dort beschrieben isoliert.

Ausbeute : 38,5 g (83 % d. Th.), Schmp. 216 °C (u. Zers.).

### Beispiel 3

5-[2-Benzylamino-4-(N-methylanilino)-5-sulfamoylphenyl]-tetrazolyl-natrium

39,3 g 2-Benzylamino-4-(N-methylanilino)-5-sulfamoylbenzolnitril (0,1 Mol) vom Schmp. 162 °C (aus Methanol) werden analog Beispiel 1 mit $HN_3$ kondensiert und nach dem Abziehen des Dimethylformamids der Rückstand aus 1n $NaHCO_3$ unter Zusatz von Aktivkohle umkristallisiert. Nach Waschen mit Wasser wird bei 100 °C getrocknet.

Ausbeute : 36 g (63 % d. Th.), Schmp. 208 °C (u. Zers.).

**Ansprüche** (für die Vertragsstaaten : BE, CH, DE, FR, GB, IT, LI, LU, NL, SE)

1. 5-Phenyltetrazol der Formel

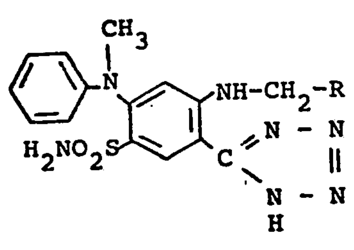

(I)

in welcher R Furyl, Thienyl oder Phenyl bedeutet, oder dessen physiologisch verträgliches Salz.

2. Verbindung gemäß Anspruch 1, in der R 2-Furyl oder 2 Thienyl ist.

3. Salz einer Verbindung gemäß Anspruch 1 mit einer basischen kaliumretinierenden Verbindung oder einem basischen Antihypertensivum.

4. Verfahren zur Herstellung eines 5-Phenyltetrazols gemäß Anspruch 1, dadurch gekennzeichnet, daß man eine Verbindung der Formel II

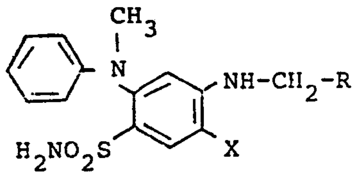

(II)

in der X Nitril, Imidoester, Amidin oder Amidrazon bedeutet, mit Stickstoffwasserstoffsäure bzw. salpetriger Säure oder einem reaktiven Derivat einer dieser Säuren umsetzt.

5. Verfahren nach Anspruch 4, dadurch gekennzeichnet, daß eine Verbindung der Formel II mit X = Nitril in einem inerten Lösungsmittel mit Stickstoffwasserstoffsäure erwärmt wird.

6. Arzneimittel bestehend aus oder enthaltend eine Verbindung gemäß Anspruch 1.

7. Verbindung gemäß Anspruch 1 zur Vervendung als Heilmittel.

**Ansprüche** (für den Vertragsstaat AT)

1. Verfahren zur Herstellung eines 5-Phenyltetrazols der Formel I

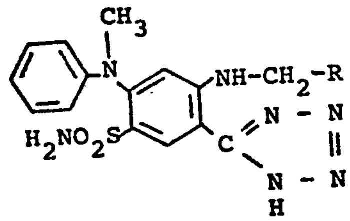

(I)

in welcher R Furyl, Thienyl oder Phenyl bedeutet, dadurch gekennzeichnet, daß man eine Verbindung der Formel II

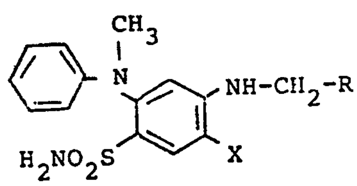

(II)

in der X Nitril, Imidoester, Amidin oder Amidrazon bedeutet, mit Stickstoffwasserstoffsäure bzw. salpetriger Säure oder einem reaktiven Derivat einer dieser Säuren umsetzt und gegebenenfalls in ihre physiologisch verträglichen Salze überführt.

2. Verfahren zur Herstellung einer Verbindung gemäß Anspruch 1, dadurch gekennzeichnet, daß R 2-Furyl oder 2 Thienyl ist.

3. Verfahren gemäß Anspruch 1, dadurch gekennzeichnet, daß man das 5-Phenyltetrazol der Formel I in das Salz mit einer basischen, kaliumretinierenden Verbindung oder einem basischen Antihypertensivum überführt.

4. Verfahren nach Anspruch 1, dadurch gekennzeichnet, daß eine Verbindung der Formel II mit X = Nitril in einem inerten Lösungsmittel mit Stickstoffwasserstoffsäure erwärmtwird.

5. Arzneimittel bestehend aus oder enthaltend eine Verbindung der Formel I gemäß Anspruch 1.

6. Verbindung der Formel I gemäß Anspruch 1 zur Vervendung als Arzneimittel.

**Claims** (for the Contracting States : BE, CH, DE, FR, GB, IT, LI, LU, NL, SE)

1. A 5-phenyltetrazole of the formula

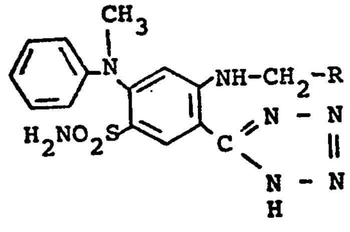

(I)

in which R denotes furyl, thienyl or phenyl, or a physiologically acceptable salt thereof.

2. A compound as claimed in claim 1, in which R is 2-furyl or 2-thienyl.

3. A salt of a compound as claimed in claim 1 with a basic, potassium-retaining compound or with a basic antihypertensive agent.

4. A process for the preparation of a 5-phenyltetrazole as claimed in claim 1, which comprises reacting a compound of the formula II

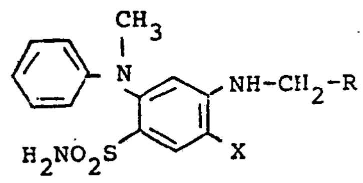

(II)

in which X denotes nitrile, imidoester, amidine or amidrazone, with hydrazoic acid or nitrous acid or a reactive derivative of one of these acids.

5. A process as claimed in claim 4, wherein a compound of the formula II in which X = nitrile is warmed with hydrazoic acid in an inert solvent.

6. A drug composed of or containing a compound as claimed in claim 1.

7. A compound as claimed in claim 1 for use as a drug.

**Claims** (for the Contracting State AT)

1. A process for the preparation of a 5-phenyltetrazole of formula I

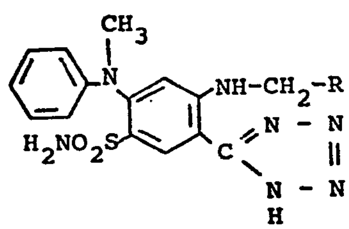

(I)

in which R denotes furyl, thienyl or phenyl, which comprises reacting a compound of the formula II

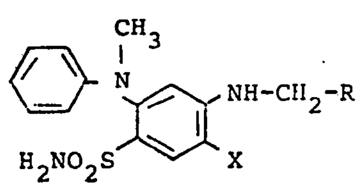

(II)

in which X denotes nitrile, imidoester, amidine or amidrazone, with hydrazoic acid or nitrous acid or a reactive derivative of one of these acids and, optionally, converting into a physiologically acceptable salt.

2. A process as claimed in claim 1, in which R is 2-furyl or 2-thienyl.

3. A process as claimed in claim 1, which comprises converting the 5-phenyltetrazole of formula I into a salt with a basic, potassium-retaining compound or with a basic antihypertensive agent.

4. A process as claimed in claim 1, wherein a compound of the formula II in which X = nitrile is warmed with hydrazoic acid in an inert solvent.

5. A drug composed of or containing a compound according to formula I of claim 1.

6. A compound of formula I of claim 1 for use as drug.

**Revendications** (pour les Etats contractants : BE, CH, DE, FR, GB, IT, LI, LU, NL, SE)

1. 5-phényltétrazole de formule

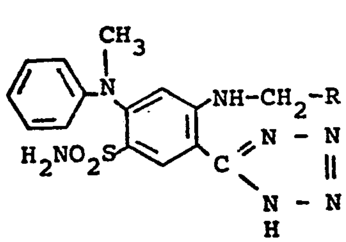

(I)

dans laquelle R représente un groupe furyle, thiényle ou phényle, ou ses sels physiologiquement acceptables.

2. Composé selon la revendication 1, dans lequel R est le groupe 2-furyle ou 2-thiényle.

3. Sel d'un composé selon la revendication 1 avec un composé basique retenant le potassium ou un antihypertenseur basique.

4. Procédé de préparation d'un 5-phényltétrazole selon la revendication 1, caractérisé en ce qu'on fait réagir un composé de formule II

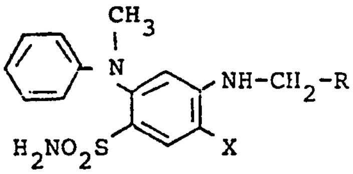

(II)

dans laquelle X représente un groupe nitrile, imidoester, amidine ou amidrazone, avec l'acide azothydrique ou l'acide nitreux ou un dérivé réactif de l'un de ces acides.

5. Procédé selon la revendication 4, caractérisé en ce qu'on chauffe un composé de formule II où X est un groupe nitrile, dans un solvant inerte, avec de l'acide azothydrique.

6. Médicament consistant en, ou contenant, un composé selon la revendication 1.

7. Composé selon la revendication 1, pour utilisation comme médicament.

**Revendications** (pour l'Etat contractant AT)

1. Procédé de préparation d'un 5-phényltétrazole de formule I

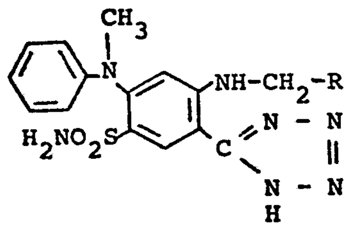

(I)

dans laquelle R représente un groupe furyle, thiényle ou phényle, caractérisé en ce qu'on fait réagir un composé posé de formule II

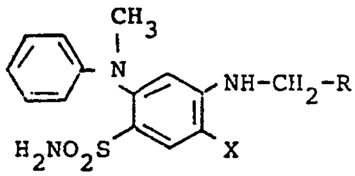

(II)

dans laquelle X représente un groupe nitrile, imidoester, amidine ou amidrazone, avec l'acide azothydrique ou l'acide nitreux ou un dérivé réactif de l'un de ces acides, et éventuellement on convertit en ses sels physiologiquement acceptables.

2. Procédé de préparation d'un composé selon la revendication 1, caractérisé en ce que R est le groupe 2-furyle ou 2-thiényle.

3. Procédé selon la revendication 1, caractérisé en ce qu'on convertit le 5-phényltétrazole de formule I en le sel avec un composé basique retenant le potassium ou avec un antihypertenseur basique.

4. Procédé selon la revendication 1, caractérisé en ce qu'on chauffe un composé de formule II où X est un groupe nitrile, dans un solvant inerte, avec de l'acide azothydrique.

5. Médicament consistant en, ou contenant, un composé de formule I selon la revendication 1.

6. Composé de formule I selon la revendication 1 pour utilisation comme médicament.